(19) Europäisches Patentamt
European Patent Office
Office européen des brevets

(11) EP 2 778 686 B1

(12) **EUROPEAN PATENT SPECIFICATION**

(45) Date of publication and mention
of the grant of the patent:
**27.07.2016  Bulletin 2016/30**

(51) Int Cl.:
***G01N 33/92*** *(2006.01)*

(21) Application number: **13159043.2**

(22) Date of filing: **13.03.2013**

(54) **Lipidomics of familial longevity**

Lipidomika mit familiärer Lebensdauer

Lipidomique de longévité familiale

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.09.2014  Bulletin 2014/38**

(73) Proprietors:
• **Academisch Ziekenhuis Leiden h.o.d.n. LUMC 2333 ZA Leiden (NL)**
• **Universiteit Leiden 2311 RA  Leiden (NL)**

(72) Inventors:
• **Slagboom, Pieternella 2333 ZA, Leiden (NL)**
• **Hankemeier, Thomas 2311 EZ, Leiden (NL)**

(74) Representative: **V.O.**
**P.O. Box 87930**
**2508 DH Den Haag (NL)**

(56) References cited:
**WO-A2-2012/164525       US-A- 5 700 447**
**US-A1- 2013 045 217**

• **ON BEHALF OF THE PROJECT GROUP AND THE LEIDEN LONGEVITY STUDY (LLS) GROUP ET AL: "Lipid metabolism in long-lived families: the Leiden Longevity Study", AGE: JOURNAL OF THE AMERICAN AGING ASSOCIATION, SPRINGER-VERLAG, DORDRECHT, NL, vol. 33, no. 2, 3 September 2010 (2010-09-03), pages 219-227, XP019909866, ISSN: 1574-4647, DOI: 10.1007/S11357-010-9172-6**
• **VANESSA GONZALEZ-COVARRUBIAS ET AL: "Lipidomics of familial longevity", AGING CELL, vol. 12, no. 3, 2 June 2013 (2013-06-02), pages 426-434, XP055067054, ISSN: 1474-9718, DOI: 10.1111/acel.12064**

**Description**

Field

This invention relates to methods for typing a sample from an individual based on a level of a lipid species in said sample, whereby said typing provides an estimate of a life expectancy for the individual.

Introduction

The human plasma lipidome is composed of thousands of different lipid species, whose diversity in function is only paralleled by its wide variation in structure (Quehenberger et al., 2010. J Lipid Res 51:, 3299-3305). The current increase of patients with lipid-related disorders has intensified the focus in lipid metabolism, dissecting it into its major classes and its connection with signaling pathways in age-related diseases (Wang et al., 2011. J Clin Endocrin & Metab 96: 885-893; Sugiyama & Agellon, 2012. Biochem Cell Biology 90: 124-141). The molecular composition and concentration of lipid species determine their cellular location, residence time, metabolism and consequently, their impact in disease and health. For example, LDL particles are the main carriers of sphingomyelins and ceramides, while ether phosphocholines are mainly present in HDL particles, partly explaining their opposing roles in atherogenesis (Nelson et al., 2006. American J Epidem163: 903-912; Yeboah et al., 2010. Arteriosclerosis, Thrombosis, and Vascular Biology. 30, 628-633).

Molecular characterization of triglyceride and sphingomyelin species has identified specific lipids underlying insulin resistance (Suhre et al., 2010. PLoS ONE. 5, e13953; Rhee et al., 2011. J Clin Invest 121: 1402-1411), Alzheimer's (Han et al., 2011. PLoS ONE. 6, e21643), and obesity (Pietilainen et al., 2007. PLoS ONE. 2, e218). Moreover, depletion of ether phosphocholine species in plasma has been associated with hypertension (Graessler et al., 2009. PLoS ONE. 4, e6261) and diabetes (Sysi-Aho et al., 2011. PLoS Comput Biol. 7, e1002257). From WO 2012/164525 it is known that several lipid species may be used as biomarkers in a method for determining a biological age of an individual. Hence, the field of metabolomics may aid to better understand health and disease by pinpointing specific metabolites and pathways involved. Up-to-date, lipidomics has been used as a powerful tool from the perspective of disease risk and treatment efficacy (Laaksonen et al., 2006. PLoS ONE. 1, e97), but not for the identification of lipid metabolites associated with longevity and healthy aging. Classical lipid parameters such as total triglyceride levels, HDL, and LDL particle size have shown to associate with human familial longevity and healthy aging (Barzilai et al., 2003. J Amer Geriat Soc 49: 76-79; Heijmans et al., 2006. PLoS Med. 3, e495). For example total triglyceride levels have shown to associate with familial longevity in women (Leiden longevity study, Vaarhorst et al., 2011. AGE: Journal of the American Aging vol.33: 219-227). Moreover, a higher ratio of monounsaturated (MUFA) over polyunsaturated (PUPA) lipids has been suggested as a marker of longevity since a small study indicated that erythrocyte membranes of offspring of nonagenarians have a higher content of MUFA and lower content of PUFA species Caprari et al., 1999. Experim Geront 34: 47-57; Puca et al., 2007.Rejuvenation Research. 11: 63-72). Therefore, lipidomics may contribute to gain new insights in the mechanisms underlying longevity and healthy aging, and may be used to differentiate between an individual with an increased life expectancy from an individual with a reduced life expectancy.

The invention therefore provides a method of typing a sample from an individual, comprising determining a level of at least one lipid species in a relevant sample from the individual, the at least one lipid species comprising ether phosphocholine (PC) (O-36:2) and/or PC (O-36:3), comparing said level with a reference, and typing said sample as a sample from an individual with an increased life expectancy if the sample has an increased level of the at least one lipid compared to the reference on the basis of said comparison. In this nomenclature, the alkyl ether linkage is represented by the "O-" prefix, whereas the numbers within parenthesis refer to the total number of carbons of the fatty acyl chains followed by the number of double bonds of all chains.

The comparison of level of the at least one lipid species provides an estimation of the expected life span of the individual which is independent of previously identified parameters such as total triglyceride concentrations.

Said at least one lipid species comprising PC (O-36:2) and/or PC (O-36:3) preferably further comprises a glycerolipid and/or a sphingolipid. The term "sphingolipid" refers to complex fomily of compounds that share a sphingoid base backbone that is synthesized from serine and a long-chain fatty acyl-CoA. Examples of these lipid species are ceramide, including sphingomyelin (ceramide phosphocholine), phosphosphingolipid, and glycosphingolipid.

Said at least one lipid species comprising PC (O-36:2) and/or PC (O-36:8) preferably further comprises a sphingomyelin (SM). Said SM preferably is SM (d18:1/14:0), SM (d18:1/15:0), SM (d18:1/16:0), SM (d18:1/17:0), SM (d18:1/18:2), SM (d18:1/21:0), SM (d18:1/23:1), and/or SM (d18:1/23:0), more preferably SM (d18:1/14:0); SM (d18:1/15:0); SM (d18:1/12:0) and/or SM (d18:1/23:1). In this nomenclature, the first number within parenthesis refers to the sphingosine followed by the number of double bonds, and the second number refers to the fatty acid followed by the number of double bonds.

It is further preferred that said at least one lipid species comprises PC (O-36:2), PC (O-36:3), SM (d18:1/14:0);

SM (d18:1/15:0); SM (d18:1/12:0) and SM (d18:1/23:1).

Said at least one lipid species furthermore preferably further comprises a glycerophospholipid and/or a glycerolipid, preferably phosphoethanolamine (PE) (38:6) and/or long-chain triglycerides (TG). In the method according to the invention a decreased level of PE (38:6) and/or long-chain triglyceride as compared to the reference indicates that the sample is from an individual with an increased life expectancy. Said TG is preferably selected from TG (52:1), TG (54:7), TG (54:6), TG (56:7), TG (56:6), and TG (57:2). More preferably, said least one lipid species two, three, four, five, or all six lipid species selected from TG (52:1), TG (54:7), TG (54:6), TG (56:7), TG (56:6), and TG (57:2).

The term "glycerolipid" refers to mono-, di- and tri-substituted glycerols. Examples of these lipid species are monoacylglycerol and triacylglycerol (also termed triglyceride).

The term "glycerophospholipid", also known as phospholipid, refers to a snglycero-3-phosphoric acid that contains at least one O-acyl, or O-alkyl, or O-alk-1'-enyl residue attached to the glycerol moiety and a polar head made of a nitrogenous base, a glycerol or an inositol unit. The "stereochemical numbering notation" (sn-notation) indicates that the hydroxyl group of the second carbon of glycerol (sn-2) is on the left on a Fischer projection, sn-1 at the top and sn-3 at the bottom. Examples of these lipid species are phosphocholine (PC), phosphoethanolamine (PE) and phosphatidylserine (PS).

Methods and systems for determining a level of said at least one lipid species in a sample are known in the art. A high resolution technique capable of resolving individual lipid species and providing structural information of these lipid species is preferred. These techniques include, for example, mass spectrometry, nuclear magnetic resonance spectroscopy, fluorescence spectroscopy or dual polarisation interferometry, a high performance separation method such as HPLC, an immunoassay such as an ELISA, and/or combinations thereof.

A preferred method comprises liquid chromatography coupled to mass spectrometry (LC-MS), for example using the method reported by Hu et al. (Hu et al. 2008. J Proteome Res 7: 4982-4991). A preferred LC system is provided by an Acquity UPLC system equipped with binary pump and autosampler (Waters, Milford MA, USA), using a T3 UPLC column (Acquity Waters). The UPLC system is coupled to a mass spectrometer, preferably a 6530 Accurate Mass QToF-LC-MS (Agilent Technologies, Santa Clara, CA, USA) equipped with electrospray ionization with jet stream nebulizer, and Agilent Mass Hunter Acquisition software.

The term "sample" refers to a sample that is isolated from the individual comprising a tissue, a cell, a fluid (e.g. urine, blood, serum, plasma, etc.), or material that isolated from a cell such as, for example, a cell or nuclear membrane of part thereof, or preferred a material that is isolated from a fluid such as a lipid fraction that is isolated from blood, preferably blood plasma.

A preferred sample is a blood sample, preferably a blood plasma sample. Methods and systems for separating blood plasma from whole blood are known in the art and include centrifugation and filtering. Serum is the liquid fraction of whole blood that is collected after the blood is allowed to clot, where after the clot is removed by centrifugation and the resulting supernatant, designated serum, is isolated. Plasma is produced when whole blood is collected in tubes comprising an anticoagulant, where after blood cells are removed by filtration or centrifugation.

A most preferred sample comprises or is a lipid extract from blood plasma. Methods for extracting lipid species from a sample, for example a plasma sample, are known in the art and include the method of Bligh and Dyer, 1959 (Can J Biochem Physiol. 37, 911-917).

The level of an individual lipid species is preferably provided as a relative ratio of said lipid species to an internal standard. For this, lipid species, for example synthetic phospholipids, are preferably added to a sample, preferably a plasma sample, as calibration and internal standards before lipid extraction. These phospholipids preferably comprise lysophosphatidylcholine (LPC), for example LPC (17:0) and/or LPC (19:0), PE (15:0/15:0), PE (17:0/17:0), PC (17:0/17:0), PC (19:0/19:0), TG (51:0) and/or TG (45:0). A relative ratio of a lipid species is obtained by dividing the area under the curve of a lipid species by the area under the curve of an internal standard that is assigned to that lipid species. For example, PC, PC-O, and SM species are preferably assigned to an internal standard PC, for example PC (17:0/17:0). A LPC species is preferably assigned to an internal standard LPC, for example LPC (19:0). A PE and/or LPE lipid species is preferably assigned to the internal standard PE, for example PE(17:0/17:0). A cholesteryl ester (ChoE), diacylglycerol and TG lipid species is preferably assigned to the internal standard TG, for example, TG (51:0). Relative ratios are preferably corrected for intra and inter batch variation prior to statistical analyses (van der Kloet et al., 2009. J Proteome Res 8: 5132-5141).

It is further preferred to run quality control samples, for example samples that are prepared from pooled plasma samples from multiple individuals, at regular intervals, for example after 20 runs, more preferred after 15 runs, more preferred after 10 runs, most preferred after 9 runs, on the high resolution technique, preferably LC-MS. In addition, randomly selected study samples are preferably analyzed as independent duplicates. It is further preferred to apply a double quality control approach by including results from lipid species for which both duplicate and QC samples show an percent relative standard deviation (%RSD) of <25%, more preferred less than 15%.

An individual according to the invention is a mammal, preferably a primate, more preferably a human. Said individual is either a male or, preferably, a female. The level of a lipid species in a sample may vary with age. It is therefore preferred that the individual, preferably a human female, is of a specified age. A human individual is, for example, younger than 30 years, between 30 and 65 years, or older than 65 years. An individual is preferably of middle age, which for a human is defined as between 30 and 65 years.

It is preferred that the reference is matched with the individual that is typed with a method of the invention. Said matching preferably includes sex- and/or age-matching, meaning that the reference is preferably obtained from an individual or a group of individuals that are of the same sex and/or of the same age group as the individual that is typed with a method of the invention.

The reference is preferably provided as a level of said at least one lipid species that was determined in a reference sample. Said reference or reference sample is preferably obtained from a corresponding tissue-, cell-, fluid-, or material sample, preferably a plasma sample, more preferably a lipid extract from blood plasma. Said reference or reference sample is preferably obtained from an individual or group of individuals of which the life expectancy is known, more preferably from an individual or a group of individuals with an average life expectancy. Said reference can be determined in parallel to the determination of the level of at least one lipid species in a relevant sample from the individual, or independent there from on a different time point but using the same of a similar high resolution technique, preferably LC-MS.

It is further preferred that the reference is a measure of the average level of said at least one lipid species that was determined in at least 2 independent individuals, more preferred at least 5 independent individuals, most preferred at least 10 independent individuals. Said independent individuals are preferably sex- and age-matched with the individual that is typed with a method of the invention. The reference is preferably stored on a computer-readable data carrier. The data carrier may include, but is not limited to, a floppy disk, an optical disk, a compact disk read-only memory (CD-ROM), a compact disk rewritable (CD-RW), a memory stick, and a magneto-optical disk.

Typing of a sample can be performed in various ways. In one method, a coefficient is determined that is a measure of a similarity or dissimilarity of a sample with said reference. A number of different coefficients can be used for determining a correlation between a level of the one or more lipid species in a sample from an individual and the reference. The result of a comparison of the determined expression levels with the expression levels of the same lipid species in a reference is preferably displayed or outputted to a user interface device, a computer readable storage medium, or a local or remote computer system. The storage medium may include, but is not limited to, a floppy disk, an optical disk, a compact disk read-only memory (CD-ROM), a compact disk rewritable (CD-RW), a memory stick, and a magneto-optical disk.

A method of the invention preferably further comprises determining the ratio of monounsaturated fatty acids (MUFA) over polyunsaturated fatty acids (PUFA) lipid species, whereby a higher ratio of MUFA over PUFA, compared to the MUFA over PUFA ratio as determined in the reference, indicates that the sample is from an individual with an increased life expectancy. Said reference is preferably obtained from an individual or group of individuals with an average life expectancy.

The invention further provides a method of prescribing a diet for an individual, comprising typing said individual according to a method of the invention, and prescribing a diet to an individual if the sample is from an individual with a decreased or average life expectancy. Said diet preferably includes a 12.5% caloric restriction. Said caloric restriction is preferably combined with an increase of 12.5% in physical exercise. Said 12.5% reduced caloric intake is on the basis of the daily caloric intake by the individual and is preferably coached by a dietician.

Figure legends

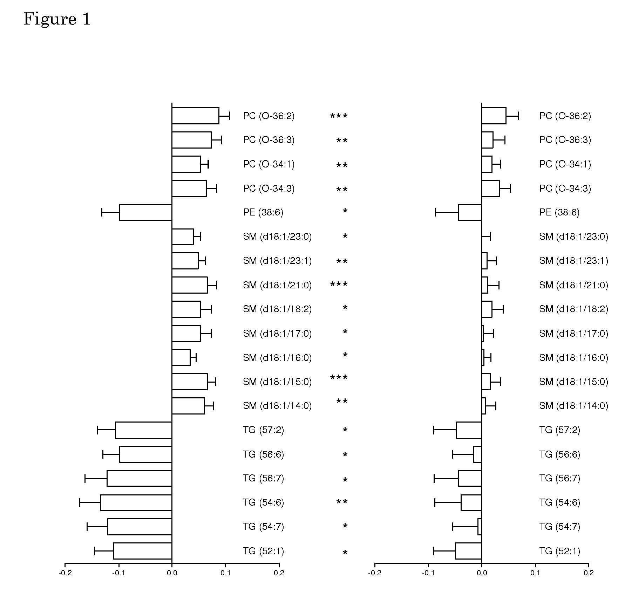

Figure 1. Differences in lipid species between offspring of nonagenarians and controls
Left, differences in females; Right, differences in males. Statistical significance was observed in females (*P<0.005; **P<0.001; ***P<0.0005). X axis depicts the effect size of levels of ln-transformed lipid species $\pm$ robust standard errors. Positive values indicate lipid levels higher in offspring. Negative values indicate lipid levels lower in offspring compared to controls.

Figure 2. MUFA-to-PUFA ratio differences in offspring of nonagenarians and controls
Adjusted mean values of MUFA-to-PUFA ratios and 95% confidence intervals; female differences P = 2.6 x 10-4; male differences P>0.05.

Figure 3. Correlation heatmap of longevity-associated lipids and clinical parameters in female offspring.

Examples

Example 1

Materials and methods

Participants. In 2001, The Leiden Longevity Study (LLS) recruited nonagenarian siblings of Caucasian descent (956 families age 90 to 104 years), 1671 of their offspring and 744 partners of the offspring as controls. The study protocol was approved by the Leiden University Medical Centre ethical committee and an informed consent was signed by all participants prior to participation. Citrate plasma was available for 1644 offspring of nonagenarians and 734 controls. After quality control, plasma lipidome measurements were considered for 2201 participants, 1526 offspring of nonagenarian siblings (59 years ± 6.7) and 675 controls (59 years ± 7.5). Table 1 lists demographic characteristics of this population and previously reported parameters such as total triglycerides, lipoprotein particle size, body mass index (BMI), LDL-C, HDL-C and prevalence of hypertension and diabetes (Mooijaart et al., 2006. PLoS Med. 3, e176; Rozing et al., 2009. Aging 1: 714-722; Westendorp et al., 2009. J Amer Geriat Soc 57: 1634-1637; Rozing et al., 2010 J Amer Geriat Soc 58: 564-569).

Lipid Profiling by LC-MS. Synthetic phospholipids, lysophosphatidylcholine LPC (17:0), LPC (19:0), PE (15:0/15:0), PE (17:0/17:0), PC (17:0/17:0), and PC (19:0/19:0) were obtained from Avanti Polar Lipids Inc. (Alabaster AL, USA), triacylglycerols TG (51:0) and TG (45:0) were obtained from Sigma Aldrich (Zwijndrecht, The Netherlands) and were added to plasma samples before lipid extraction as calibration and internal standards. Lipids from plasma aliquots (30 μL) were extracted according to the method of Bligh and Dyer with slight modifications (Bligh and Dyer, 1959. Can J Biochem Physiol 37: 911-917).

Lipidomic analysis was performed using an optimized version of the method reported by C.Hu et al (Hu et al., 2008. J Proteome Res 7: 4982-4991). Briefly, lipid extracts spiked with calibration and internal standards were separated by reverse phase ultra-high pressure liquid chromatography coupled to mass spectrometry (UPLC-MS), using an Acquity UPLC system equipped with binary pump and autosampler at 15 °C (Waters, Milford MA, USA). Lipids were separated in a T3 UPLC column 1.8μm, 2.1 x 100mm (Acquity Waters) at 55°C using a dynamic gradient from 68% to 3% mobile phase A to mobile phase B in 17 minutes, followed by equilibration for a total run of 20 minutes. Mobile phase A consisted of water and acetonitrile (40:60 %V/V), supplemented with 10mM ammonium formate. Mobile phase B contained acetonitrile, isopropanol (10:90 %V/V), and 10mM ammonium formate. All solvents were UPLC grade (Biosolve, Valkenswaard, The Netherlands). The UPLC was coupled to a QToF mass spectrometer, 6530 Accurate Mass QToF-LC-MS (Agilent Technologies, Santa Clara, CA, USA) equipped with electrospray ionization with jet stream nebulizer, and Agilent Mass Hunter Acquisition software. Data were acquired using the following settings: gas temperature 325°C, nozzle voltage 1000V, gas flow 10 L/min, sheath gas flow 12 L/min, Vcap 3500V, mass range 450 - 1600 m/z, in positive ion mode. The method was validated using plasma obtained with sodium citrate. Validation parameters were: linearity LPC (19:0), r2>0.99; PC (17:0/17:0), r2>0.97; PE (17:0/17:0), r2>0.98; TG (45:0), r2>0.99; repeatability and reproducibility, RSD <15%. Two freeze-thaw cycles did not alter validation parameters (RSD<15%). After data acquisition, we used a targeted method and data mining to assess the relative levels of 150 lipids using MassHunter Quantitative Analysis Software (v.B.04.00, Agilent Technologies, 2008).

Lipid Nomenclature. Lipids names and abbreviations were assigned according to Lipid Maps nomenclature (http://www.lipidmaps.org). The analytical method determined total lipid composition as number of carbon atoms and double bonds without specifying the location of double bonds or the stereochemistry of the acyl chains, and differentiated the alkyl-acyl (ether) - from the diacyl- subclasses. The following accepted abbreviations were used: phosphocholines, PC; sphingomyelins, SM; triglycerides, TG; phosphoethanolamines, PE. The alkyl ether linkage is represented by the "O-" prefix e.g. PC (O-34:1), TG (O-50:2) the numbers within parenthesis refer to the total number of carbons of the fatty acyl chains followed by the number of double bonds of all the chains.

Lipid Quantification. Relative ratios (RR) of each lipid species were obtained by dividing the area under the curve of each lipid by the area under the curve of its assigned internal standard. PC, PC-O, and SM species were assigned to the internal standard PC (17:0/17:0); LPC species - to the internal standard LPC (19:0); PE and LPE species - to the internal standard PE (17:0/17:0); cholesteryl ester (ChoE), diacylglycerol, and TG species - to the internal standard TG (51:0). Relative ratios (RR) were corrected for intra and inter batch variation prior to statistical analyses (van der Kloet et al., 2009. J Proteome Res 8: 5132-5141). Quality control: quality control samples (QC) prepared with pooled plasma from the same cohort were included every 9 injections. In addition, randomly selected study samples (15%) were analyzed as independent duplicates. A double quality control approach was applied to include lipid species that were accurately measured throughout the study by considering only lipids species for which both, duplicate and QC samples showed an RSD<15% (n=111) or RSD<25% (n=128).

Statistical Analyses. Most lipid species showed a right-skewed distribution thus, data were logarithmically transformed to approximate normality before statistical analyses.

For comparisons between offspring and controls a linear regression model was fitted with lipid species as dependent variables and offspring/control status as a fixed factor. Data analyses were stratified by sex to assess sex-specific differences. Lipid species strongly correlated with BMI and age hence, these parameters were used as covariates. Also, a term for batch was included in the model to account for analytical batch effects.

To assess whether the association of lipid species linked to familial longevity was independent of total triglycerides, logistic regression analyses were performed in females with offspring/control status as the dependent variable and total triglycerides, age, BMI, and batch as independent variables. Family relationships were taken into account by including in the regression model a term to determine robust standard errors using the sandwich estimator which considered data from one same family as a group. MUFA-to-PUFA ratios were calculated for the lipid species that associated with longevity by adding levels of all MUFA lipids (lipids with one double bond in any acyl chain) and the resulting value was divided by the sum of all PUFA lipids (species with two or more double bonds in their acyl chains) i.e. $\sum$MUFA/$\sum$PUFA. Lipid species, PC (O-36:2) and TG (57:2) were not considered in this calculation because the unknown location of the double bond made their classification as polyunsaturated or monounsaturated uncertain. MUFA-to-PUFA ratios were logarithmically transformed for further analyses. To test the effect of age after 55 years a linear regression model was fitted with age before or after menopause (>55 or $\leq$55 years) as a dependent variable separately in offspring and controls. This study represents the first attempt to associate lipidomic profiles and familial longevity. Therefore, our exploratory approach defined statistical significance at an arbitrary threshold of P-value $\leq$ 0.005 for all analyses. Statistical analyses were performed using STATA 11.0 (StataCorp LP, TX, USA, 2009) and R statistical programming system (http://www.r-project.org/). Graphics were created with GraphPad Prism (version 4.0, La Jolla, CA, USA).

Results

We successfully obtained lipidomic profiles consisting of 128 different lipid species in 1526 offspring and 675 controls. Population characteristics in Table 1 show that offspring and controls were similar in terms of age, BMI, and most classical lipid parameters except for total triglycerides and the TG-HDL-C ratio whose values were higher in controls (P<0.005). Also, compared to the offspring of nonagenarians, controls were more likely to present diabetes and hypertension. Demographic differences between males and females are presented in Table S1.

Relative lipid levels were calculated for a total of 128 lipids from six different classes: TG (n=52), SM (n=17), PE (n=7), cholesterylesters (n=4), lysophosophocholines (n=16), and PC (n=32). We compared the relative levels of these 128 lipids between offspring of nonagenarians and controls to identify lipid species that associate with familial longevity. In males, lipidomic differences between offspring and controls did not reach statistical significance. However, in women we identified nineteen lipids that significantly associated with familial longevity (Figure 1). Relative levels of ether PC and SM species were higher (3.5 - 8.7%) while, long-chain triglycerides and PE (38:6) were lower (9.4 - 12.4%) in the offspring of nonagenarians compared to controls (Table S2). TG (54:6) and PC (O-36:2) species showed the largest differences between offspring and controls with 12.4% lower and with 8.7% higher levels in female offspring. In males, we obtained similar results for ether PC and TG species, but effect sizes between offspring and controls were smaller than in females and differences did not reach statistical significance (Table S3).

Total triglycerides have been previously reported as a determinant of familial longevity in women of the Leiden Longevity Study (Vaarhorst et al., 2011. AGE. 33: 219-227). Regression analyses accounting for total triglycerides showed that the association with familial longevity remained significant for six lipid species (P<0.005). Female longevity in LLS families was associated with ether PC (O-36:2), PC (O-36:3), SM (d18:1/14:0), SM (d18:1/15:0), SM (d18:1/21:0), and SM (d18:1/23:1) independent of total TG (P<0.005, Table S4). In addition, we investigated whether lipid levels in female offspring and controls were affected by age. Nine of the 19 lipid species either increased or decreased with age, but most of them were not affected by age after 55 years, except for SM (d18:1/23:1) and TG (54:6) which showed differential levels before and after 55 years in female offspring but not in controls (Tables 2 and 3).

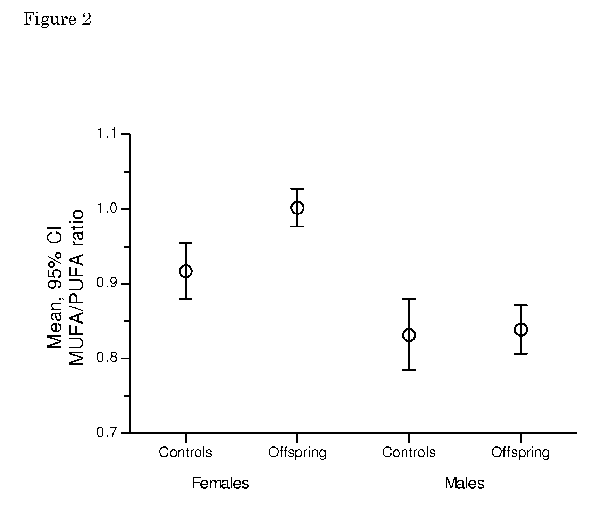

Female offspring of nonagenarians showed a higher MUFA-to-PUFA ratio Differences in content of polyunsaturated (PUFA) and monounsaturated (MUFA) lipids determine membrane peroxidation and the MUFA-to-PUFA ratio has been suggested as a marker of longevity (Pamplona et al., 2000. Mechanisms Ageing Develop 112: 169-183; Caprari et al., 1999. Experim Geront 34: 47-57). Therefore, we determined differences in the MUFA-to-PUFA ratio between offspring and controls for each one of the nineteen lipid species that associated with familial longevity. Offspring of nonagenarians displayed a higher MUFA-to-PUFA ratio compared to controls (Figure 2) and these differences were statistically significant in females only. Compared to controls, female offspring showed a 9.2% higher MUFA-to-PUFA ratio (MUFA-to-PUFA ratio controls: 0.917, CI: 0.880 - 0.955 versus MUFA-to-PUFA ratio offspring: 1.001, CI: 0.977 - 1.027; P-value = 2.6 x 10-4).

Effect of sex and age on lipids associated with familial longevity

We observed several lipidomic differences between sexes. All long-chain TG species were lower in females compared to males particularly TG (52:1), TG (54:6), and TG (57:2), while PE (38:6), ether PC, and SM species were higher in females (P<0.005). The largest differences between sexes were observed for all SM, PC (O-34:1), and PC (O-34:3) species, which were higher in women (Table 2).

Next, we explored the effect of chronological age on these 19 lipid species. We detected a significant association between age and levels of 9 of the 19 lipids. Most ether PC species showed a negative association while all other longevity-associated lipids displayed a positive association with age, except for SM (d18:1/18:2), and (d18:1/23:0) which were not significantly affected by age. The most significant effect of age was observed for ether PC (O-34:3) and (O-36:3) showing negative and TG (52:1), (54:7), and (57:2) showing positive associations with age (Table 2 and Table S5). In addition, Pearson correlation coefficients indicated a linear correlation with age for 10 and 13 lipid species in female controls and offspring, respectively (R<0.25, P<0.005; Table S6). When considering age after 55 years, only two lipid species, SM (d18:1/23:1) and TG (54:6), showed an effect of age (Table 3). A summary of linear parameters of the effect of age after 55 years on these 19 lipid species is listed in Table S6. Our results indicate that higher levels of ether PC and lower levels of PE (38:6) and TG species are representative of a youthful profile, supporting the notion that offspring of nonagenarians have a healthy attenuated aging process (Westendorp et l., 2009. J Amer Geriat Soc 57: 1634-1637).

Correlation between longevity-associated lipids, classical lipids, and clinical parameters

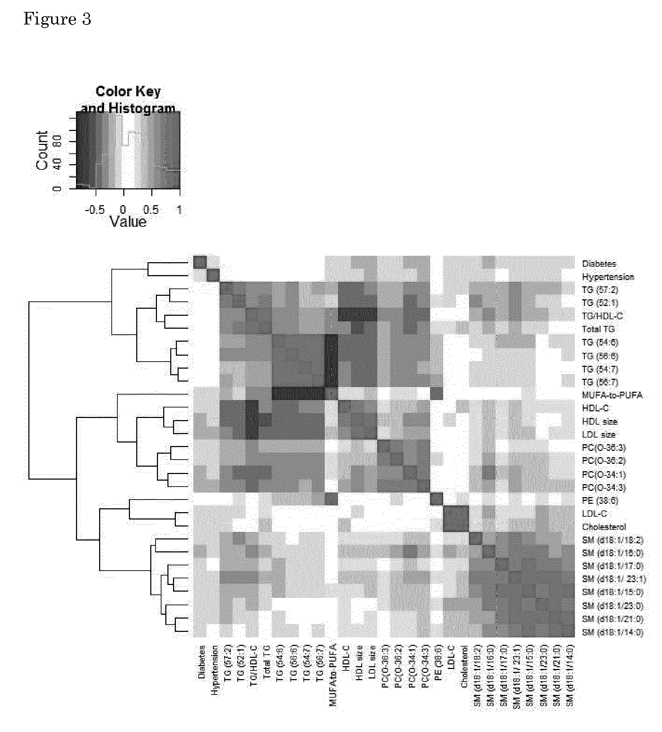

We investigated potential correlations between the 19 longevity-associated lipids and classical lipid parameters including, total triglycerides, HDL-C, LDL-C, lipoprotein particle size, the TG-HDL-C and MUFA-to-PUFA ratios, and the prevalence of hypertension or diabetes. Besides the expected correlations among lipids of similar classes shown as highly correlated clusters in Figure 3, we also detected lipidomic correlations among lipid species not previously reported. First, PC (O-34:1) was positively correlated to SM (d18:1/16:0), and negatively to TG (57:2), (54:6), and (52:1). Similarly, SM (d18:1/23:1) and (d18:1/18:2) negatively correlated with TG (57:2) and (52:1).

Among classical lipid parameters, total triglycerides negatively correlated with HDL-C, lipoprotein particle size, SM (d18:1/23:1), SM (d18:1/16:0), and most ether PC, particularly PC (O-34:3) and PC (O-34:1). In addition, HDL particle size was positively correlated to PC (O-34:3) and PC (O-34:1), while LDL and HDL particle size inversely correlated with TG (54:6), TG (56:6), and to the TG/HDL-C ratio.

The MUFA-to-PUFA ratio showed an inverse correlation to PE (38:6) and several TG species, and was positively correlated to lipoprotein particle size, HDL-C, SM (d18:1/15:0), ether PC (O-36:2), and PC (O-34:1).

Since recent investigations have pinpointed specific lipid species linked to hypertension and diabetes we sought to identify correlations between the 19 lipid species and the prevalence of these diseases. Ether PC (O-34:1), PC (O-34:3), SM (d18:1/16:0), and SM (d18:1/23:1) showed a prominent inverse correlation with diabetes type 2 and in a similar manner PC (O-34:1), PC (O-34:3), and PC (O-36:1) with hypertension.

Example 2

In the family based Leiden Longevity Study long-lived Caucasian siblings were recruited together with their offspring and the partners thereof.

Families were recruited if at least two long-lived siblings were alive and fulfilled the age-criterion of 89 years or older for males and 91 year or older for females, representing less than 0.5 % of the Dutch population in 2001. In total 944 long-lived proband siblings were included with a mean age of 93 (89-103) years, 1671 offspring of a mean age 59 (34-80) years and 744 partners of a mean age of 59 (30-79) years.

Inclusion criteria are middle-aged (≤75 years) couples consisting of offspring from long-lived siblings and his/her current partner or in incidental case as single participant with a BMI ≥23 and ≤35 kg/m2. Exclusion criteria are type I or type II diabetes (on diabetic medication); individuals who have lost or gained ≥3 kg over the past 6 months; individuals engaged in heavy/intensive physical activity (top sport or physically heavy work); any disease or condition that seriously affects body weight and/or body composition including active types of cancer, heart failure (NYHA III/VI), COPD (GOLD III/VI); recent (3 months prior to intervention) immobilisation for longer than 1 week; psychiatric or behavioural problems (eg, history or clinical manifestation of any eating disorders, vegan dietary lifestyle, major depression); medication: thyroid medication, immunosuppressive drugs (e.g. prednisone, methotrexat, biologicals (TNF-alpha antagonists etc); and concurrent participation in any other intervention study or weight management program.

Exclusion criteria for biopsy are the use of anticoagulantia (e.g. coumarines, carbaspirin calcium).

Exclusion criteria for MR imaging (3 tesla and 7 tesla) are claustrophobia; pacemakers and defibrillators; nerve stimulators; intracranial clips; intraorbital or intraocular metallic fragments; cochlear implants; ferromagnetic implants (e.g. thoracic implant for scoliosis); inability to lie supine during for 45 minutes; and not having a general practitioner.

Sample size calculation

The sample size calculation is primarily based on the results of the CALERIE study, in which a decline in insulin concentration of 21.1% (from 9.8 to 7.69 µIU/mL) was observed after 3 months of a combined caloric restriction and exercise intervention (Heilbronn et al., 2006. JAMA 295:1539-48).

In the subsample of offspring and partners within the Leiden Longevity study that likely fulfills to our main study criteria the following LN insulin concentrations were observed:

Offspring: LN insulin mean 2.84, SD 0.82; Spouses: LN insulin mean 2.89, SD 0.83.

The first part of the power analysis is based on these findings and the assumption that the spouses in our study will show a decline in insulin concentrations similar to the one in the CALERIE study (21%). With a power of 80%, a significance level ($\alpha$) of 0.05, a 0.61 (=21%) decline in LN(insulin) and an SD of the change of 1.17 (sqrt(2*0.832)), the required sample size is 61 subjects per group (so 61 couples), as calculated by the program G-Power 3.0.10 (independent t-test). Taking into account a dropout rate of 10 %, we would aim to include 70 couples in the intervention.

However, note that in this calculation we actually assume that the insulin levels of the offspring will not decline. This is not very likely, as the offspring is also subjected to the intervention, to be able to investigate the impact of the intervention on several of their physiological and molecular parameters as well. Actually, we do not have enough subjects in LLS to carry out a study investigating the full interaction effect, i.e. does the intervention work better in spouses than in offspring. Yet, with these numbers (70 spouses) and SD's, we can pick up a change of 0.36 in insulin concentration in spouses using a paired t-test (comparing before and after), which is the power analyses relevant to the primary objective, and feasible given the CALERIE results.

TREATMENT OF SUBJECTS

Subjects are treated during 3 months intervention with 25% lowered energy expenditure by 12.5% caloric restriction and 12.5% more exercise.

METHODS

Main study parameter/endpoint are a change in fasting insulin levels. Secondary study parameters/endpoints (if applicable) are a change in several blood parameters related to glucose and lipid metabolism, and plasma thyroid markers; 24 hour glucose monitoring + glucose load; muscle biopsy (changes in mRNA expression, protein amounts, signalling pathways); fat biopsy (changes in mRNA expression, protein amounts, signalling pathways); artrose biomarkers in urine; blood pressure; anthropometrics (weight, height and waist circumference); body composition (lean mass (kg and %), appendicular lean mass, fat mass); muscle strength and gait; resting metabolic rate and VO2max (suboptimal); MR imaging (leg, brain, cartilage of the knee); cognitive functioning; mood; quality of life; sleep; and hunger, appetite and eating behavior.

Other study parameters (if applicable) include physical activity level and type of activity (resting, sitting, walking, running); health and lifestyle questionnaire (smoking behaviour, etc); compliance; and number of adverse and serious adverse events.

A screening is used to assess if the individuals fulfill to the in- and exclusion criteria and consists of one screening session at the participant's home. The screening will take approximately 30 minutes and comprises:

• Medical questionnaire

The medical questionnaire will provide information on medical status and medication use. Diabetic patients, individuals with any disease or condition that seriously affects body weight and/or body composition and individuals with psychiatric or behavioural problems will be excluded.

• Blood glucose

A finger prick is a non-invasive way to determine blood glucose levels. Subjects will be measured in the morning after an overnight fast. Subjects with a fasting blood glucose level $\geq$ 7,0 mmol, indicating glucose intolerance and/or type 2 diabetes according to the World Health Organization criteria of 1999 (Alberti and Zimmet 1998), will be excluded.

• BMI

Height and weight will be measured to determine BMI, because subjects with a BMI ≤ 23 or ≥35 kg/m2 will be excluded.

• Physical activity level.

The habitual amount of physical activity will be estimated with a physical activity questionnaire (IRAQ)

Pre-baseline

The pre-baseline assessments will take approximately 1 hour and include:

• Habitual dietary intake

To determine the habitual dietary pattern of the participants they have to fill in an online food frequency questionnaire (FFQ). The FFQ is a valid and reliable method for assessing average consumption of food intake. In the FFQ subjects are asked how often over the past month they have eaten a pre-specified number of 150 food items. Frequency of consumption can be indicated per day, per week, per month, or never. The FFQ will be filled in at home and checked later for completion and quality of the information by a dietician. The guidelines for the 12.5% caloric restriction will be based on the results of the FFQ and will be made by the dietician in consultation with the subjects.

• Physical activity measurement

To assess the habitual amount of physical activity all individuals have to wear an accelerometer (GENEA) on the wrist and ankle. This is a small wrist-worn device comparable with a normal wristwatch, to wear for seven days at home. After a week, participants can send the accelerometer back by mail. The accelerometer quantitatively measures physical activity and health behaviour. The guidelines for the 12.5% increase in physical activity will be based on the data collected with the accelerometer, in addition with the data collected with the physical activity questionnaire performed at screening. The guidelines will be made by a physiotherapist in consultation with the subjects.

Baseline and end measurements
Late afternoon/ evening measurements

These measurements will take approximately 3.5 hours in total and include:

• Blood pressure

After a 10-minute rest, blood pressure will be measured 5 times with a 2-minute interval on the dominant arm by trained staff members using a validated blood pressure device. The average of the last 4 measurements is recorded in the CRF.

• Anthropometry

Weight, height, leg length and waist and hip circumference will be measured. Weight will be measured to the nearest 0.5 kg with the person dressed in light clothing and without shoes. Height will be measured to the nearest 0.1 cm with the person in standing position and wearing no shoes.
Waist circumference will be measured in duplicate to the nearest 0.1 cm at the midpoint between the lowest rib and the top of the iliac crest with a non-elastic tape and in standing position.

• Bioelectrical impedance analysis (BIA)

Body composition will also be assessed with bioelectrical impedance analysis, using the In-Body (720) body composition analyzer. This device can measure total body water, extracellular water and intracellular water. The subject should be in a supine position for four minutes before the start of the measurement. Throughout the measurement the subjects arms and legs should be slightly apart and the subject should remain motionless. The measurement will last approximately one minute.

• DEXA scan

Body composition will be assessed by Dual Energy X-ray Absorptiometry (DEXA) scan. With a DEXA scan lean body mass, skeletal muscle mass and body fat percentage will be determined on a whole body level as well as for specific regions. The DEXA scan will be done in the LUMC and is a simple, non-invasive procedure requiring no injections, sedation, special diet, or any other advanced preparation and is completely safe. The radiation is comparable with 3 hours skiing on a sunny day when the sky is clear. At the beginning of the procedure subjects will be asked to lie down on a scan table and they need to remain motionless during the measurements. As the scanner moves, a dual energy beam (0.03 mrem) passes through the targeted skeletal muscle section and is measured by a detector. This procedure is repeated until the whole body is scanned and takes approximately 15 minutes.

• Resting metabolic rate

Resting metabolic rate will be measured by means of indirect calorimetrie, using the ventilated hood system. The subjects will be placed under the ventilated hood, while lying on a bed in a quiet room for 30 minutes. The subject must stay awake. This measurement will take about 30 minutes. Measurements of VO2 and VCO2 will be made every minute. Subsequently, glucose antilipid oxidation can be calculated with the following equation:

Glucose oxidation = 4.57 VCO2 - 3.23VO2 - 2.6N
Lipid oxidation = 1.69 VO2 - 1.69VCO2 - 2.03N

Resting energy expenditure (REE) is calculated from:

$$REE = 3.91\ VO2 + 1.10\ VCO2 - 1.93N,$$

Protein disappearance is ignored since the error thus introduced on the measurement of energy expenditure is negligible.

• Mood

The short form of the Profile of Mood States (POMS-SF) (Curran et al., 1995. Psychological assessment 7:80-83) and Center for Epidemiologic Studies Depression Scale (CES-D) (Radloff, 1977. Applied Psychological Measurement 1:385-401) questionnaire will be used to assess mood and depressive symptoms.

• Quality of Life

Quality of life of the subjects will be assessed using the 12-Item Short Form Health Survey (SF-12) (Ware et al., 1996. Medical care 34::220-33).

• Cognitive performance

Cognitive function tests are performed by a trained research assistant under supervision of a neuropsychologist. The measurements focus on the domains: memory, interference, reaction time and speed. The following tests will be included:
MMSE: The Mini Mental State Examination (MMSE) is a short questionnaire to examine various cognitive functions, including orientation to time and place, verbal imprinting, attention, short-term memory, language, and constructive skills. It consists of 11 short questions and tasks that can be completed in 5-10 minutes (Folstein et al., 1975. J Psychiatr Res 12:189-98).
Picture Learning Test (PLT): testing immediate and delayed recall. The main outcome parameters will be the accumulated number of recalled pictures over the three learning trials and the number of pictures recalled after 20 minutes.

Duration: 10-15 minutes
Focus: (immediate) memory and interference
Wechsler Digit Span Task (forward and backward): The examiner verbally presents digits at a rate of one per second. The forward test requires the participant to repeat the digits verbatim. The backward test requires the participant to repeat the digits in reverse order. The number of digits increases by one, until the participant consecutively fails two trials of the same digit span length.
Duration: 5 minutes
Focus: memory

Letter Symbol Substitution Test:

Nine different letters are assigned a unique number (1-9) in a key at the top of the form. The participants are presented with a random series of letters in cells and are instructed to add the corresponding digit to the letters. The number of correctly copied corresponding digits in 90 seconds will be recorded.

Duration: 3 minutes
Focus: Information processing speed, visuomotor coordination and selective attention.

The Stroop Test: A combination of words "red", "blue", "yellow" and "green" is printed in red, blue, yellow and green ink interchangeably. The participant is asked to name the colours of the words (which do not necessarily correspond to each other) and reading the words, which lead to a so called "colour-word interference effect".

Duration: 7 minutes
Focus: interference

Speed test on computer

• Sleep

Subjects will fill out the Munich Chronotype Questionnaire about their sleeping behaviour.

• Hunger

At baseline and at the end the Dutch Eating Behaviour questionnaire will be distributed for completion, which included questions on eating behaviour, relevant to the program. During the contacts with the dietician a short questionnaire on hunger and appetite will be asked.

• MR imaging (3 and 7 Tesla)

We will use proton magnetic resonance spectroscopy (1H-MRS, 7 Tesla) of the leg for measurement of intramyocellular lipid content. Participants will undergo an MRS measurement of intramyocellular and extramyocellular lipid content in the anterior tibial muscle (fig 1). Participants will be placed in the MRI-scanner in feet-first position. After initial imaging, a voxel will be placed at the level of the anterior tibial muscle of one of the lower limbs, after which MRS-imaging will be completed.

A 7T MRI will be performed providing information on the cerebral microvasculature and brain (resting state fMRI, ASL, MRA) at baseline and of the cartilage of the knee at the endline measurements. Total scanning time will take approx. 1 hour.

• Grip strength and gait

Three consecutive measures of handgrip strength (kgf) at both hands will be recorded to the nearest 0.5 kg using a hand dynamometer (Jamar). Gait speed and balance will be measured using the short physical performance test (SPPB).

• 24 hour glucose monitoring with glucose load

This involves the application of small-sized, minimally invasive and easy to wear glucose monitor, which is to be worn for 5 days. During these days the subject has to measure blood glucose by means of a finger prick 4 times a day. While wearing the device all daily activities can be performed as usual. During the test day the subjects will get a 75 g glucose load, to assess glucose tolerance. Blood (9 ml at each time point) will be drawn prior to (t=0) and at 30, 60, 90 and 120 minutes after the glucose load.

One day of wearing the CGM yields 288 glucose measurements, resulting in:

i) high statistical power to detect differences and trends over time,

ii) detailed information on a number of physiologically novel, relevant parameters such as maximum peak height, slope of peak rise after a meal, fasting glucose levels. Using continuous glucose measurements with 288 data points per 24 hours, more characteristics of the 24-hour glucose rhythm can be detected, such as maximum peak height, mean during 24 hours, difference between highest and lowest point, speed of increase after oral glucose challenge, etc

• Artrose markers in urine

Morning measurements in fasting state

These measurements will take approximately 1 hour in total.

• Blood sample

A fasted blood sample (95 ml) will be collected by venapunction for determination of blood concentrations related to glucose and lipid metabolism, plasma thyroid markers, isolation of peripheral blood mononuclear cells (PBMCs) and RNA determination.

• Muscle biopsy

A muscle biopsy will be taken from the musculus vastus lateralis, 10 cm of the cranial side of the patella on the lateral side of the upper leg under local anaesthesia. A small incision (about 5 mm) is made, after this the biopsy needle (about 3 mm thick) will be inserted to obtain the muscle. Incision site is closed with surgical tape and a compression bandage is applied.

The muscle biopsies will immediately be frozen in liquid nitrogen and then be stored at -80°C for subsequent analysis to determine mRNA expression, protein amounts and to specify signaling pathways stimulated by the intervention.

• Fat biopsy

The skin of the abdomen is cleansed and a local anesthetic is used to numb the area. A needle is inserted through the skin and into the fat pad under the skin. A small core of the fat pad is removed with the needle. The biopsies will be stored at -80°C for subsequent analysis to determine mRNA expression and to specify signaling pathways stimulated by the intervention.

The intervention

During a 13 weeks of intervention participants will follow a dietary regime including a 12.5% reduction in energy intake and a 12.5% increase in energy expenditure through physical activity, leading 25% caloric restriction. The program will be delivered through intensive counseling by trained dieticians and physiotherapists. To fulfill this program participants have to adhere to previously determined individual, tailored guidelines and record their eating behaviour and physical activity in a diary. During the intervention the accelorometer will also be used to determine physical activity. The guidelines for the 12.5% dietary restriction will be individually based on FFQ data performed prior to the intervention and according to the 'Dutch Guidelines for a healthy diet ('Richtlijnen Goede Voeding' (RGV)) (Health Council of the Netherlands 2006). The guidelines for the 12.5% increase in physical activity will be based on data derived from the accelerometer and physical activity questionnaire and matched to the subjects' preference and capabilities.

To check and stimulate adherence participants will weekly be in contact with a dietician and a physiotherapist (alternating), who will check their body weight (weekly) and waist circumference (monthly). These weekly contacts will be at the participants' home or by phone (according to an alternating schedule) and will also be used to check adherence to the guidelines and to discuss practical problems and solutions.

Withdrawal of individual subjects

Subjects can leave the study at any time for any reason if they wish to do so without any consequences. The investigator can decide to withdraw a subject from the study for urgent medical reasons.

Replacement of individual subjects after withdrawal

Our intention is to start with sufficient couples, taking into account a 10% dropout rate, rather than replacing dropouts. However, in case subjects withdraw during their first 3 weeks of intervention, new couples will replace them when sufficient couples are available and fulfill to the eligibility criteria.

SAFETY REPORTING

Section 10 WMO event

In accordance to section 10, subsection 1, of the WMO, the investigator will inform the subjects and the reviewing accredited METC if anything occurs, on the basis of which it appears that the disadvantages of participation may be significantly greater than was foreseen in the research proposal. The study will be suspended pending further review by the accredited METC, except insofar as suspension would jeopardize the subjects' health. The investigator will take care that all subjects are kept informed.

Adverse and serious adverse events

Adverse events are defined as any undesirable experience occurring to a subject during the study, whether or not considered related to [the investigational product / the experimental treatment]. All adverse events reported spontaneously by the subject or observed by the investigator or his staff will be recorded.
A serious adverse event is any untoward medical occurrence or effect that at any dose results in death; is life threatening (at the time of the event); requires hospitalisation or prolongation of existing inpatients' hospitalisation; results in persistent or significant disability or incapacity; is a congenital anomaly or birth defect; is a new event of the trial likely to affect the safety of the subjects, such as an unexpected outcome of an adverse reaction, lack of efficacy of an IMP used for the treatment of a life threatening disease, major safety finding from a newly completed animal study, etc.
All SAEs will be reported through the web portal ToetsingOnline to the accredited METC that approved the protocol, within 15 days after the sponsor has first knowledge of the serious adverse reactions.
SAEs that result in death or are life threatening should be reported expedited. The expedited reporting will occur not later than 7 days after the responsible investigator has first knowledge of the adverse reaction. This is for a preliminary report with another 8 days for completion of the report.

Follow-up of adverse events

All adverse events will be followed until they have abated, or until a stable situation has been reached. Depending on the event, follow up may require additional tests or medical procedures as indicated, and/or referral to the general physician or a medical specialist.

STATISTICAL ANALYSIS

Descriptive statistics

For continuous variables, differences between baseline and end of offspring and spouses will be analyzed with a paired t-test. For categorical variables chi-square analysis will be performed.

Statistical analysis

Basically, changes from baseline to the end of the study within offspring and within spouses will be analyzed using a paired t-test. If necessary, variables will be normalized by log transformation. For additional analyses, e.g. differences between offspring and spouses the study will be analyzed with an independent samples t-test, and Analysis of Covariance (ANCOVA) to adjust for differences in age and sex between offspring and spouses. The ANCOVA (also for repeated measurements) will be programed using random effects models, as this is statistically more efficient (missing data can be handled). All intervention analyses will be carried out according to intention to treat. Next, markers of compliance (subjective assessment by dietician, body weight reduction, reduction in self-reported energy intake, participation in planned sports activities) will be used in additional analysis.
A 2-sided P-value of <0.05 will be considered statistically significant. Statistical analysis will be performed using PASW Statistics.

ETHICAL CONSIDERATIONS

Regulation statement

This study will be conducted according to the principles of the Declaration of Helsinki and in accordance with the Medical Research Involving Human Subjects Act (WMO).

Recruitment and consent

Potential offspring and controls (children from the long-living subjects and their partners) will be contacted by mail. After two weeks this letter will be followed up by a phone call. When they are interested to participate they are asked to complete an initial general and medical questionnaire on eligibility criteria of the study. When both partners fulfill to these criteria, they will be visited by a member of the research team for screening measurements. Before they are screened they have to complete and sign an informed consent form. They will be given ample time (minimum 1 week) and information to decide whether or not to participate in the study.

Benefits and risks assessment, group relatedness
Benefits for the subjects

The subjects will receive a final report of the tests that will be performed. In this report, their own results will be presented. Moreover, the final group results will be presented after the results have been published. Subjects will benefit from a personalized, tailored, dietary and physical activity advice by a dietician and physiotherapist. If adherence to these guidelines is good they will lose weight and improve several metabolic parameters related to healthy aging.

Risks for the subjects

The risks involved in participating in this experiment are minimal. A venapunction is comparable to a normal blood drawn and the only risk is that of a small local hematoma. The incision made for obtaining the fat and the muscle biopsies will be done by an experienced physician and will heal completely.

Table 1 Demographics of the study population

|  | Controls (675) | Offspring (1526) |
|---|---|---|
| N | males (285), females (390) | males (714), females (812) |
| Age (years) | 58.8 ± 7.4 (30.2 - 79.2) | 59.4 ± 6.6 (33.6 - 80.3) |
| BMI (kg/m2) | 25.3 ± 3.6 (25.3 - 25.9) | 25.8 ± 3.6 (25.2 - 25.5) |
| Total TG (mmol/L) | 1.94 ± 1.37 (1.81 - 2.03) | 1.76 ± 1.05 (1.69 - 1.80) |
| HDL-C (mmol/L) | 1.43 ± 0.465 (1.40 - 1.47) | 1.45 ± 0.448 (1.43 - 1.48) |
| LDL-C (mmol/L) | 3.36 ± 0.938 (3.29 - 3.43) | 3.34 ± 0.987 (3.29 - 3.39) |
| HDL particle size (nm) | 9.01 ± 0.509 (8.99 - 9.10) | 9.05 ± 0.507 (9.03 - 9.08) |
| LDL particle size (nm) | 21.1 ± 0.838 (21.09 - 21.21) | 21.3 ± 0.826 (21.2 - 21.3) |
| TG/HDLC ratio | 1.66 ± 1.54 (1.54 - 1.78) | 1.45 ± 1.27 (1.39 - 1.52) |
| Diabetes type 2 (%)[a] | 47 (8.2) | 56 (4.3) |
| Hypertension (%)[b] | 166 (28.7) | 288 (22.3) |
| MUFA-to-PUFA ratio[b] | 0.881 ± 0.37 (0.851 - 0.911) | 0.928 ± 0.380 (0.907 - 0.948) |
| Data are reported as mean values ± SD followed by range values in parentheses. [a]Values are number of cases followed by percentage in parenthesis. [b]Values are adjusted means from the regression model ± SD followed by range values between parenthesis. | | |

Table 2 Effect of age (males and females) and sex in lipid species that associated with familial longevity

| Lipid | Adjusted Mean[a] | Effect of Age | | Effect of Sex | |
|---|---|---|---|---|---|
| | | Beta ± SE[b] | P-value[c] | Beta ± SE[d] | P-value[c] |
| PC (O-34:3) | 0.736 | -0.067 ± 0.010 | $1.0 \times 10^{-11}$ | -0.134 ± 0.012 | $<10^{-20}$ |
| PC (O-34:1) | 0.41 | -0.021 ± 0.008 | $7.0 \times 10^{-3}$ | -0.121 ± 0.010 | $<10^{-20}$ |
| PC (O-36:3) | 0.176 | -0.041 ± 0.011 | $1.3 \times 10^{-4}$ | -0.096 ± 0.013 | $1.3 \times 10^{-12}$ |
| PC (O-36:2) | 0.122 | -0.029 ± 0.011 | $1.1 \times 10^{-2}$ | -0.098 ± 0.014 | $6.5 \times 10^{-12}$ |
| PE (38:6) | 0.819 | 0.046 ± 0.019 | $1.3 \times 10^{-2}$ | -0.221 ± 0.026 | $4.3 \times 10^{-17}$ |
| SM (d18:1/14:0) | 0.362 | 0.039 ± 0.009 | $2.2 \times 10^{-5}$ | -0.154 ± 0.011 | $<10^{-20}$ |
| SM (d18:1/15:0) | 0.229 | 0.050 ± 0.009 | $5.0 \times 10^{-8}$ | -0.177 ± 0.011 | $<10^{-20}$ |
| SM (d18:1/16:0) | 6.624 | 0.012 ± 0.006 | $3.9 \times 10^{-2}$ | -0.969 ± 0.008 | $<10^{-20}$ |
| SM (d18:1/17:0) | 0.085 | 0.056 ± 0.010 | $1.0 \times 10^{-8}$ | -0.190 ± 0.012 | $<10^{-20}$ |
| SM (d18:1/18:2) | 0.023 | 0.003 ± 0.010 | $7.8 \times 10^{-1}$ | -0.274 ± 0.013 | $<10^{-20}$ |
| SM (d18:1/21:0) | 0.282 | 0.021 ± 0.011 | $3.3 \times 10^{-2}$ | -0.210 ± 0.013 | $<10^{-20}$ |
| SM (d18:1/23:1) | 0.501 | 0.033 ± 0.008 | $8.1 \times 10^{-5}$ | -0.251 ± 0.010 | $<10^{-20}$ |
| SM (d18:1/23:0) | 0.74 | 0.002 ± 0.008 | $7.5 \times 10^{-1}$ | -0.144 ± 0.010 | $<10^{-20}$ |
| TG (52:1) | 4.065 | 0.073 ± 0.018 | $7.2 \times 10^{-5}$ | 0.225 ± 0.026 | $2.4 \times 10^{-17}$ |
| TG (54:7) | 0.376 | 0.063 ± 0.021 | $3.0 \times 10^{-3}$ | 0.144 ± 0.028 | $4.7 \times 10^{-7}$ |
| TG (54:6) | 1.38 | 0.043 ± 0.023 | $5.7 \times 10^{-2}$ | 0.222 ± 0.031 | $8.6 \times 10^{-13}$ |
| TG (56:7) | 1.021 | 0.052 ± 0.020 | $1.0 \times 10^{-2}$ | 0.192 ± 0.028 | $1.8 \times 10^{-11}$ |
| TG (56:6) | 1.116 | 0.041 ± 0.018 | $2.3 \times 10^{-2}$ | 0.157 ± 0.024 | $4.9 \times 10^{-11}$ |
| TG (57:2) | 1.216 | 0.059 ± 0.018 | $1.0 \times 10^{-3}$ | 0.219 ± 0.025 | $1.7 \times 10^{-18}$ |

[a] Back-transformed adjusted means of relative lipid levels; [b] Beta, effect size of age in 10 years and robust standard error; [d] Beta, effect size of sex and robust standard error; negative values indicate lipid levels lower in males, positive values indicate lipid levels higher in males; [c] Nominal P-value.

Table 3

| Effect of age after 55 years on levels of lipid species that associated with familial longevity | | | | |
|---|---|---|---|---|
| | Controls | | Offspring | |
| | Beta ± SE[a] | P-value[b] | Beta ± SE[a] | P-value[b] |
| PC (O-34:3) | 0.037 ± 0.322 | 0.909 | 0.034 ± 0.369 | 0.927 |
| PC (O-34:1) | -0.384 ± 0.303 | 0.206 | -0.124 ± 0.236 | 0.600 |
| PC (O-36:3) | 0.015 ± 0.372 | 0.969 | -0.352 ± 0.372 | 0.345 |
| PC (O-36:2) | 0.029 ± 0.386 | 0.940 | -0.718 ± 0.428 | 0.094 |
| PE (38:6) | -0.900 ± 0.680 | 0.186 | 0.602 ± 0.652 | 0.356 |
| SM (d18:1/14:0) | -0.051 ± 0.278 | 0.854 | 0.524 ± 0.288 | 0.069 |
| SM (d18:1/15:0) | -0.368 ± 0.296 | 0.215 | 0.408 ± 0.274 | 0.136 |
| SM (d18:1/16:0) | -0.049 ± 0.221 | 0.825 | 0.344 ± 0.185 | 0.064 |
| SM (d18:1/17:0) | -0.118 ± 0.329 | 0.719 | -0.002 ± 0.448 | 0.996 |
| SM (d18:1/18:2) | -0.589 ± 0.404 | 0.146 | 0.093 ± 0.341 | 0.786 |

(continued)

| Effect of age after 55 years on levels of lipid species that associated with familial longevity | | | | |
|---|---|---|---|---|
| | Controls | | Offspring | |
| | Beta ± SE[a] | P-value[b] | Beta ± SE[a] | P-value[b] |
| SM (d18:1/21:0) | -0.058 ± 0.319 | 0.855 | 0.418 ± 0.315 | 0.186 |
| SM (d18:1/23:1) | -0.169 ± 0.283 | 0.550 | 0.682 ± 0.226 | 0.003 |
| SM (d18:1/23:0) | 0.197 ± 0.255 | 0.440 | 0.630 ± 0.238 | 0.008 |
| TG (52:1) | -0.050 ± 0.691 | 0.942 | 1.40 ± 0.632 | 0.027 |
| TG (54:7) | -0.672 ± 0.786 | 0.393 | 1.24 ± 0.637 | 0.052 |
| TG (54:6) | -0.360 ± 0.793 | 0.650 | 1.86 ± 0.690 | 0.005 |
| TG (56:7) | -0.686 ± 0.727 | 0.346 | 1.10 ± 0.656 | 0.094 |
| TG (56:6) | -0.699 ± 0.609 | 0.252 | 1.18 ± 0.537 | 0.028 |
| TG (57:2) | -0.779 ± 0.633 | 0.219 | 0.828 ± .544 | 0.129 |

[a] Beta, effect size of age after 55 years and robust standard error; negative values indicate lipid levels decrease and positive values indicate lipid levels increase after 55 years; [b] Nominal P-value.

Supplemental Table 1. Demographics of the Study Population Stratified by Sex

| | Males | | Females | |
|---|---|---|---|---|
| | Controls | Offspring | Controls | Offspring |
| N | 285 | 714 | 390 | 812 |
| Age (years) | 61.4 ± 7.5 | 59.3 ± 6.6 | 56.9 ± 6.8 | 59.3 ± 6.5 |
| BMI (kg/m2) | 25.8 ± 3.2 | 25.7 ± 2.9 | 25.4 ± 3.8 | 25.0 ± 4.0 |
| TG (mmol/L) | 2.21 ± 1.68 | 2.00 ± 1.18 | 1.74 ± 1.06 | 1.55 ± 0.860 |
| HDL-C (mmol/L) | 1.23 ± 0.351 | 1.27 ± 0.366 | 1.55 ± 0.491 | 1.59 ± 0.459 |
| LDL-C (mmol/L) | 3.32 ± 0.937 | 3.29 ± 0.932 | 3.39 ± 0.939 | 3.38 ± 1.03 |
| HDL particle size (nm) | 8.78 ± 0.419 | 8.81 ± 0.441 | 9.18 ± 0.506 | 9.25 ± 0.469 |
| LDL particle size (nm) | 20.8 ± 0.775 | 20.9 ± 0.784 | 21.4 ± 0.797 | 21.5 ± 0.750 |
| TG/HDLC ratio | 2.06 ± 1.78 | 1.83 ± 1.49 | 1.37 ± 1.27 | 1.11 ± 0.909 |
| Diabetes type 2 (%)[a] | 27 (9.5) | 31 (3.8) | 20 (5.1) | 25 (3.1) |
| Hypertension (%)[a] | 70 (24.6) | 135 (16.6) | 97 (24.8) | 153 (18.7) |
| MUFA-to-PUFA ratio[b] | 0.832 ± 0.382 | 0.839 ± 0.401 | 0.917 ± 0.350 | 1.00 ± 0.341 |

Data are reported as mean values ± SD unless otherwise specified. [a]Values are number of cases followed by percentage in parenthesis [b]Values are adjusted means from the regression model ± SD.

Supplemental Table 2. Lipid species that associated with familial longevity in females

| Lipid | Controls[a] | Offspring[a] | Beta ± SE[b] | P-value[c] |
|---|---|---|---|---|
| PC (O-34:3) | 1.474 | 1.572 | 0.064 ± 0.019 | $6.1 \times 10^{-4}$ |
| PC (O-34:1) | 0.618 | 0.651 | 0.053 ± 0.015 | $2.7 \times 10^{-4}$ |
| PC (O-36:3) | 0.275 | 0.296 | 0.073 ± 0.020 | $2.0 \times 10^{-4}$ |
| PC (O-36:2) | 0.183 | 0.199 | 0.087 ± 0.020 | $1.4 \times 10^{-5}$ |

(continued)

| Lipid | Controls[a] | Offspring[a] | Beta $\pm$ SE[b] | P-value[c] |
|---|---|---|---|---|
| PE (38:6) | 1.197 | 1.085 | $-0.098 \pm 0.033$ | $3.2 \times 10^{-3}$ |
| SM (d18:1/14:0) | 0.351 | 0.373 | $0.061 \pm 0.016$ | $1.4 \times 10^{-4}$ |
| SM (d18:1/15:0) | 0.209 | 0.223 | $0.066 \pm 0.016$ | $2.8 \times 10^{-5}$ |
| SM (d18:1/16:0) | 6.943 | 7.184 | $0.034 \pm 0.011$ | $2.8 \times 10^{-3}$ |
| SM (d18:1/17:0) | 0.072 | 0.076 | $0.054 \pm 0.019$ | $3.6 \times 10^{-3}$ |
| SM (d18:1/18:2) | 0.035 | 0.037 | $0.054 \pm 0.019$ | $5.0 \times 10^{-3}$ |
| SM (d18:1/21:0) | 0.316 | 0.337 | $0.066 \pm 0.017$ | $8.9 \times 10^{-5}$ |
| SM (d18:1/23:1) | 0.573 | 0.601 | $0.049 \pm 0.014$ | $5.6 \times 10^{-4}$ |
| SM (d18:1/23:0) | 0.852 | 0.886 | $0.040 \pm 0.014$ | $4.9 \times 10^{-3}$ |
| TG (52:1) | 1.626 | 1.457 | $-0.110 \pm 0.035$ | $2.0 \times 10^{-3}$ |
| TG (54:7) | 0.165 | 0.147 | $-0.121 \pm 0.038$ | $1.4 \times 10^{-3}$ |
| TG (54:6) | 0.510 | 0.447 | $-0.133 \pm 0.040$ | $9.7 \times 10^{-4}$ |
| TG (56:7) | 0.470 | 0.416 | $-0.122 \pm 0.041$ | $3.3 \times 10^{-3}$ |
| TG (56:6) | 0.521 | 0.472 | $-0.098 \pm 0.031$ | $1.7 \times 10^{-3}$ |
| TG (57:2) | 0.545 | 0.490 | $-0.106 \pm 0.031$ | $6.9 \times 10^{-4}$ |

[a] Back-transformed adjusted means of relative lipid levels; [b] Beta, effect size of lipid levels and robust standard error. Positive values indicate higher levels in offspring of nonagenarians and negative values indicate lower levels in offspring compared to controls; Nominal P-value.

Supplemental Table 3. Lipid species that associated with familial longevity in males

| Lipid | Controls[a] | Offspring[a] | Beta $\pm$ SE[b] | P-value[c] |
|---|---|---|---|---|
| PC (O-34:3) | 1.221 | 1.262 | $0.033 \pm 0.021$ | 0.111 |
| PC (O-34:1) | 0.505 | 0.515 | $0.019 \pm 0.016$ | 0.239 |
| PC (O-36:3) | 0.252 | 0.257 | $0.021 \pm 0.023$ | 0.378 |
| PC (O-36:2) | 0.165 | 0.173 | $0.045 \pm 0.024$ | 0.067 |
| PE (38:6) | 0.493 | 0.471 | $-0.044 \pm 0.042$ | 0.305 |
| SM (d18:1/14:0) | 0.399 | 0.402 | $0.007 \pm 0.020$ | 0.715 |
| SM (d18:1/15:0) | 0.260 | 0.264 | $0.016 \pm 0.020$ | 0.401 |
| SM (d18:1/16:0) | 7.677 | 7.708 | $0.004 \pm 0.013$ | 0.765 |
| SM (d18:1/17:0) | 0.104 | 0.105 | $0.003 \pm 0.019$ | 0.895 |
| SM (d18:1/18:2) | 0.038 | 0.039 | $0.019 \pm 0.021$ | 0.380 |
| SM (d18:1/21:0) | 0.397 | 0.402 | $0.011 \pm 0.021$ | 0.614 |
| SM (d18:1/23:1) | 0.706 | 0.713 | $0.010 \pm 0.018$ | 0.591 |
| SM (d18:1/23:0) | 1.020 | 1.020 | $2.0 \times 10^{4} \pm 0.017$ | 0.991 |
| TG (52:1) | 1.931 | 1.839 | $-0.049 \pm 0.042$ | 0.239 |
| TG (54:7) | 0.252 | 0.250 | $-0.007 \pm 0.047$ | 0.881 |
| TG (54:6) | 1.245 | 1.198 | $-0.039 \pm 0.049$ | 0.436 |
| TG (56:7) | 0.643 | 0.616 | $-0.043 \pm 0.047$ | 0.354 |

(continued)

| Lipid | Controls[a] | Offspring[a] | Beta $\pm$ SE[b] | P-value[c] |
|---|---|---|---|---|
| TG (56:6) | 0.604 | 0.574 | -0.051 $\pm$ 0.031 | 0.197 |
| TG (57:2) | 0.659 | 0.628 | -0.048 $\pm$ 0.038 | 0.216 |

[a] Back-transformed adjusted means of relative lipid levels; Beta, effect size of lipid levels and robust standard error; positive values indicate higher levels in offspring of nonagenarians and negative values indicate lower levels in offspring compared to controls; [c] Nominal P-value.

Supplemental Table 4. Lipid species that associated with familial longevity in females independent of total triglycerides

| Lipid | Beta $\pm$ SE[a] | P-value[b] |
|---|---|---|
| PC (O-34:3) | 0.719 $\pm$ 0.274 | 0.009 |
| PC (O-34:1) | 0.958 $\pm$ 0.352 | 0.006 |
| PC (O-36:3) | 0.754 $\pm$ 0.244 | 0.002 |
| PC (O-36:2) | 0.928 $\pm$ 0.241 | $1.1 \times 10^{-4}$ |
| PE (38:6) | -0.308 $\pm$ 0.156 | 0.049 |
| SM (d18:1/14:0) | 1.172 $\pm$ 0.307 | $1.3 \times 10^{-4}$ |
| SM (d18:1/15:0) | 1.176 $\pm$ 0.303 | $1.0 \times 10^{-4}$ |
| SM (d18:1/16:0) | 1.031 $\pm$ 0.428 | 0.016 |
| SM (d18:1/17:0) | 0.618 $\pm$ 0.371 | 0.096 |
| SM (d18:1/18:2) | 0.646 $\pm$ 0.266 | 0.015 |
| SM (d18:1/21:0) | 1.096 $\pm$ 0.280 | $9.0 \times 10^{-4}$ |
| SM (d18:1/23:1) | 0.924 $\pm$ 0.322 | 0.004 |
| SM (d18:1/23:0) | 0.886 $\pm$ 0.330 | 0.007 |
| TG (52:1) | -0.163 $\pm$ 0.185 | 0.379 |
| TG (54:7) | -0.293 $\pm$ 0.142 | 0.039 |
| TG (54:6) | -0.277 $\pm$ 0.142 | 0.060 |
| TG (56:7) | -0.281 $\pm$ 0.175 | 0.107 |
| TG (56:6) | -0.305 $\pm$ 0.187 | 0.104 |
| TG (57:2) | -0.280 $\pm$ 0.166 | 0.092 |

[a] Beta, effect size of lipid levels and robust standard error from logistic regression analysis; positive values indicate higher levels in the offspring of nonagenarians and negative values indicate lower levels in offspring compared to controls; [b] Nominal P-value.

Supplemental Table 5. Effect of age in lipid species that associated with familial longevity in males versus females

| Lipid | Females N = 1202 | | Males N = 999 | |
|---|---|---|---|---|
|  | Beta $\pm$ SE[b] | P-value[c] | Beta $\pm$ SE[b] | P-value[c] |
| PC (O-34:3) | -0.076 $\pm$ 0.013 | $9.07 \times 10^{-09}$ | -0.060 $\pm$ 0.014 | $2.7 \times 10^{-5}$ |
| PC (O-34:1) | 0.034 $\pm$ 0.010 | $9.10 \times 10^{-04}$ | -0.009 $\pm$ 0.011 | 0.427 |
| PC (O-36:3) | -0.049 $\pm$ 0.015 | $7.22 \times 10^{-04}$ | -0.037 $\pm$ 0.015 | 0.015 |

(continued)

| Lipid | Females N = 1202 | | Males N = 999 | |
|---|---|---|---|---|
| | Beta ± SE[b] | P-value[c] | Beta ± SE[b] | P-value[c] |
| PC (O-36:2) | -0.044 ± 0.016 | $5.00 \times 10^{-03}$ | -0.017 ± 0.016 | 0.281 |
| PE (38:6) | 0.042 ± 0.023 | $7.17 \times 10^{-02}$ | 0.058 ± 0.029 | 0.049 |
| SM (d18:1/14:0) | 0.052 ± 0.012 | $1.62 \times 10^{-05}$ | 0.017 + 0.013 | 0.179 |
| SM (d18:1/15:0) | 0.064 ± 0.012 | $9.47 \times 10^{-08}$ | 0.029 ± 0.013 | 0.036 |
| SM (d18:1/16:0) | 0.020 ± 0.008 | $9.18 \times 10^{-03}$ | -0.001 ± 0.001 | 0.929 |
| SM (d18:1/17:0) | 0.082 ± 0.014 | $2.46 \times 10^{-09}$ | 0.020 ± 0.014 | 0.135 |
| SM (d18:1/18:2) | 0.003 ± 0.014 | $8.43 \times 10^{-01}$ | -0.002 ± 0.014 | 0.883 |
| SM (d18:1/21:0) | 0.042 ± 0.013 | $1.04 \times 10^{-03}$ | -0.009 ± 0.015 | 0.566 |
| SM (d18:1/23:1) | 0.051 ± 0.011 | $2.85 \times 10^{-06}$ | 0.006 ± 0.012 | 0.616 |
| SM (d18:1/23:0) | 0.022 ± 0.010 | $3.11 \times 10^{-02}$ | -0.024 ± 0.012 | 0.024 |
| TG (52:1) | 0.098 ± 0.024 | $5.42 \times 10^{-05}$ | 0.052 ± 0.028 | 0.065 |
| TG (54:7) | 0.109 ± 0.027 | $6.55 \times 10^{-05}$ | 0.023 ± 0.034 | 0.493 |
| TG (54:6) | 0.097 ± 0.028 | $6.82 \times 10^{-04}$ | -0.090 ± 0.037 | 0.813 |
| TG (56:7) | 0.082 ± 0.027 | $2.52 \times 10^{-03}$ | 0.025 ± 0.032 | 0.431 |
| TG (56:6) | 0.088 ± 0.023 | $1.30 \times 10^{-04}$ | -0.009 ± 0.028 | 0.735 |
| TG (57:2) | 0.071 ± 0.023 | $2.45 \times 10^{-03}$ | 0.052 ± 0.027 | 0.053 |

[a] Beta, effect size of lipid levels and robust standard error from linear regression analysis; positive values indicate higher levels in the offspring of nonagenarians and negative values indicate lower levels in offspring compared to controls; [b] Nominal P-value.

Supplemental Table 6. A: Linear parameters between age and levels of lipid species that associated with familial longevity in females

| | Offspring | | | | | | | | |
|---|---|---|---|---|---|---|---|---|---|
| | All ages (812) | | | <55 years (200) | | | >55 years (612) | | |
| | B | Slope | R | B | Slope | R | B | Slope | R |
| PC (O-34:3) | 0.227 | -0.0097 | -0.224* | 0.135 | -0.0078 | -0.089 | 0.169 | -0.0088 | -0.154* |
| PC (O-34:1) | -0.500 | -0.0058 | -0.174* | -0.444 | -0.0067 | -0.108 | -0.568 | -0.0047 | -0.104 |
| PC (O-36:3) | -1.179 | -0.0078 | -0.166* | -0.971 | -0.0116 | -0.117 | -1.323 | -0.0055 | -0.090 |
| PC (O-36:2) | -1.695 | -0.0077 | -0.163* | -1.214 | -0.0168 | -0.175 | -1.932 | -0.0040 | -0.064 |
| PE (38:6) | 0.199 | 0.0029 | 0.038 | -0.455 | 0.0160 | 0.096 | 0.148 | 0.0036 | 0.037 |
| SM (d18:1/14:0) | -0.744 | 0.0041 | 0.114* | -1.141 | 0.0117 | 0.157 | -0.616 | 0.0021 | 0.043 |
| SM (d18:1/15:0) | -1.446 | 0.0057 | 0.157* | -1.669 | 0.0097 | 0.134 | -1.261 | 0.0028 | 0.058 |
| SM (d18:1/16:0) | 2.027 | 0.0004 | 0.016 | 1.821 | 0.0042 | 0.086 | 2.165 | -0.0017 | -0.050 |
| SM (d18:1/17:0) | -2.367 | 0.0067 | 0.151* | -2.075 | 0.0003 | 0.002 | -2.078 | 0.0022 | 0.044 |
| SM(d18:1/18:2) | -3.128 | 0.0016 | 0.039 | -3.104 | 0.0009 | 0.011 | -3.012 | -0.0002 | -0.004 |
| SM (d18:1/21:0) | -0.889 | 0.0029 | 0.073 | -1.131 | 0.0073 | 0.089 | -0.714 | 0.0001 | 0.002 |
| SM (d18:1/23:1) | -0.346 | 0.0031 | 0.092 | -0.772 | 0.0110 | 0.162 | -0.090 | -0.0009 | -0.021[a] |

(continued)

| | Offspring | | | | | | | | |
|---|---|---|---|---|---|---|---|---|---|
| | All ages (812) | | | <55 years (200) | | | >55 years (612) | | |
| | B | Slope | R | B | Slope | R | B | Slope | R |
| SM (d18:1/23:0) | 0.066 | 0.0005 | 0.016 | -0.301 | 0.0073 | 0.105 | 0.330 | -0.0036 | -0.083 |
| TG (52:1) | 1.280 | 0.0104 | 0.122* | 0.124 | 0.0329 | 0.191* | 1.525 | 0.0065 | 0.057 |
| TG (54:7) | -1.236 | 0.0114 | 0.135* | -2.256 | 0.0312 | 0.176 | -1.016 | 0.0079 | 0.071 |
| TG (54:6) | 0.003 | 0.0122 | 0.134* | -1.485 | 0.0410 | 0.219* | 0.374 | 0.0063 | 0.052[a] |
| TG (56:7) | -0.104 | 0.0101 | 0.120* | -0.894 | 0.0251 | 0.140 | 0.209 | 0.0051 | 0.047 |
| TG (56:6) | -0.081 | 0.0118 | 0.165* | -1.083 | 0.0313 | 0.205* | 0.101 | 0.0089 | 0.094 |
| TG (57:2) | -0.057 | 0.0079 | 0.096* | -0.793 | 0.0223 | 0.136 | 0.035 | 0.0064 | 0.058 |

B, line intercept at age = 0; R, Pearson correlation coefficient; * Pearson correlation coefficient, P<0.005 and slopes are different from zero, P<0.005. [a]
Differences in slopes between offspring and controls were statistical significant, P<0.005.

Supplemental Table 6, B,

| | Controls | | | | | | | | |
|---|---|---|---|---|---|---|---|---|---|
| | All ages (390) | | | <55 years (147) | | | >55 years (243) | | |
| | B | Slope | R | B | Slope | R | B | Slope | R |
| PC (O-34:3) | 0.074 | -0.0081 | -0.191* | -0.385 | 0.0017 | 0.028 | -0.348 | -0.0016 | -0.023 |
| PC (O-34:1) | -0.662 | -0.0039 | -0.124 | -0.728 | -0.0021 | -0.044 | -1.112 | 0.0032 | 0.065 |
| PC (O-36:3) | -1.387 | -0.0052 | -0.122 | -1.976 | 0.0074 | 0.108 | -1.961 | 0.0037 | 0.057 |
| PC (O-36:2) | -2.022 | -0.0035 | -0.081 | -2.584 | 0.0086 | 0.138 | -2.555 | 0.0048 | 0.070 |
| PE (38:6) | 0.036 | 0.0072 | 0.099 | 0.897 | -0.0106 | -0.093 | -0.003 | 0.0082 | 0.070 |
| SM (d18:1/14:0) | -0.979 | 0.0074 | 0.214* | -1.059 | 0.0091 | 0.171 | -1.110 | 0.0094 | 0.171 |
| SM (d18:1/15:0) | -1.615 | 0.0077 | 0.220* | -1.590 | 0.0075 | 0.147 | -1.958 | 0.0132 | 0.225* |
| SM (d18:1/16:0) | 1.873 | 0.0025 | 0.104 | 1.755 | 0.0051 | 0.142 | 1.706 | 0.0051 | 0.127 |
| SM (d18:1/17:0) | -2.620 | 0.0103 | 0.261* | -2.663 | 0.0113 | 0.195 | -2.782 | 0.0128 | 0.200* |
| SM (d18:1/18:2) | -3.114 | 0.0007 | 0.016 | -2.755 | -0.0065 | -0.099 | -3.344 | 0.0045 | 0.067 |
| SM (d18:1/21:0) | -1.174 | 0.0070 | 0.191* | -1.382 | 0.0115 | 0.219 | -1.440 | 0.0111 | 0.182* |
| SM (d18:1/23:1) | -0.634 | 0.0074 | 0.236* | -0.654 | 0.0080 | 0.171 | -0.824 | 0.0104 | 0.204*[a] |
| SM (d18:1/23:0) | -0.202 | 0.0046 | 0.156* | -0.430 | 0.0094 | 0.221 | -0.233 | 0.0050 | 0.101 |
| TG (52:1) | 1.371 | 0.0109 | 0.139* | 1.587 | 0.0063 | 0.052 | 1.537 | 0.0083 | 0.065 |
| TG (54:7) | -1.241 | 0.0138 | 0.161* | -0.721 | 0.0032 | 0.025 | -1.393 | 0.0164 | 0.116 |
| TG (54:6) | 0.230 | 0.0110 | 0.120 | 0.662 | 0.0020 | 0.015 | 0.302 | 0.0100 | 0.066[a] |
| TG (56:7) | -0.026 | 0.0111 | 0.120 | 0.638 | -0.0026 | -0.022 | -0.048 | 0.0117 | 0.072 |
| TG (56:6) | 0.202 | 0.0088 | 0.124 | 0.973 | -0.0072 | -0.071 | 0.274 | 0.0080 | 0.067 |
| TG (57:2) | -0.026 | 0.0092 | 0.141* | 0.610 | -0.0038 | -0.039 | -0.170 | 0.0117 | 0.109 |

B, line intercept at age = 0; R, Pearson correlation coefficient; * Pearson correlation coefficient, P<0.005 and slopes are different from zero, P<0.005. [a]
Differences in slopes between offspring and controls were statistical significant, P<0.005.

**Claims**

1. A method of typing a sample from a mammalian individual, comprising:

   determining a level of at least one lipid species in a relevant sample from the individual, the at least one lipid species comprising ether phosphocholine (PC) (O-36:2) and/or PC (O-36:3); comparing said level with a reference; and typing said sample as a sample from an individual with an increased life expectancy if the sample has an increased level of the at least one lipid compared to the reference on the basis of said comparison.

2. The method according to claim 1, wherein said at least one lipid species comprising PC (O-36:2) and/or PC (O-36:3) further comprises at least one of sphingomyelin (SM) (d18:1/14:0); SM (d18:1/15:0); SM (d18:1/12:0) and/or SM (d18:1/23:1) and wherein an increased level of PC (O-36:2), PC (O-36:3), SM (d18:1/14:0); SM (d18:1/15:0); SM (d18:1/12:0) and/or SM (d18:1/23:1) compared to the level of the respective lipid species in the reference, indicates that the sample is from an individual with an increased life expectancy.

3. The method according to claim 2, wherein said at least one lipid species comprises PC (O-36:2), PC (O-36:3), SM (d18:1/14:0); SM (d18:1/15:0); SM (d18:1/12:0) and SM (d18:1/23:1).

4. The method according to any one of claims 1-3, wherein said at least one lipid species further comprises phosphoethanolamine (PE) (38:6) and/or long-chain triglyceride (TG) and wherein a decreased level of PE (38:6) and/or long-chain triglyceride as compared to the reference indicates that the sample is from an individual with an increased life expectancy.

5. The method according to any one of claims 1-4, wherein the sample is a blood sample.

6. The method according to any one of claims 1-5, wherein the individual is a human.

7. The method according to any one of claims 1-6, wherein the individual is a female.

8. The method according to any one of claims 1-7, wherein the individual is of middle age.

9. The method according to any one of the previous claims, wherein the reference is a measure of the average level of said at least one lipid species in at least 10 independent individuals.

10. The method according to claim 9, wherein said at least 10 independent individuals are sex- and age-matched with the individual.

11. The method according to any one of the previous claims, wherein the level of said at least one lipid species in a reference sample is stored on a computer-readable data carrier.

12. The method according to any one of the previous claims, further comprising determining the ratio of monounsaturated fatty acids (MUFA) over polyunsaturated fatty acids (PUFA) lipid species, whereby a higher ratio of MUFA over PUFA, compared to the MUFA over PUFA ratio as determined in the reference, indicates that the sample is from an individual with an increased life expectancy.

**Patentansprüche**

1. Verfahren zur Typisierung einer Probe von einem Säugetier-Individuum, umfassend:

   Bestimmen eines Spiegels von mindestens einer Lipidspezies in einer relevanten Probe von dem Individuum, die mindestens eine Lipidspezies umfassend Etherphosphocholin (PC) (O-36:2) und/oder PC (O-36:3); Vergleichen des Spiegels mit einer Referenz; und Typisieren der Probe als eine Probe von einem Individuum mit einer erhöhten Lebenserwartung, wenn die Probe einen erhöhten Spiegel des mindestens einen Lipids verglichen mit der Referenz auf der Grundlage des Vergleichs aufweist.

2. Verfahren nach Anspruch 1, wobei die mindestens eine Lipidspezies umfassend PC (O-36:2) und/oder PC (O-36:3) ferner mindestens eines von Sphingomyelin (SM) (d18:1/14:0); SM (d18:1/15:0); SM (d18:1/12:0) und/oder SM (d18:1/23:1) umfasst und wobei ein erhöhter Spiegel von PC (O-36:2), PC (O-36:3), SM (d18:1/14:0); SM (d18:1/15:0); SM (d18:1/12:0) und/oder SM (d18:1/23:1), verglichen mit dem Spiegel der entsprechenden Lipidspezies in der Referenz, darauf hinweist, dass die Probe von einem Individuum mit einer erhöhten Lebenserwartung stammt.

3. Verfahren nach Anspruch 2, wobei die mindestens eine Lipidspezies PC (O-36:2), PC (O-36:3), SM (d18:1/14:0); SM (d18:1/15:0); SM (d18:1/12:0) und SM (d18:1/23:1) umfasst.

4. Verfahren nach einem der Ansprüche 1-3, wobei die mindestens eine Lipidspezies ferner Phosphoethanolamin (PE) (38:6) und/oder langkettiges Triglycerid (TG) umfasst und wobei ein verminderter Spiegel von PE (38:6) und/oder langkettigem Triglycerid, verglichen mit der Referenz, darauf hinweist, dass die Probe von einem Individuum mit einer erhöhten Lebenserwartung stammt.

5. Verfahren nach einem der Ansprüche 1-4, wobei die Probe eine Blutprobe ist.

6. Verfahren nach einem der Ansprüche 1-5, wobei das Individuum ein Mensch ist.

7. Verfahren nach einem der Ansprüche 1-6, wobei das Individuum eine Frau ist.

8. Verfahren nach einem der Ansprüche 1-7, wobei das Individuum mittleren Alters ist.

9. Verfahren nach einem der vorhergehenden Ansprüche, wobei die Referenz ein Maß des durchschnittlichen Spiegels von mindestens einer Lipidspezies in mindestens 10 unabhängigen Individuen ist.

10. Verfahren nach Anspruch 9, wobei die mindestens 10 unabhängigen Individuen geschlechts- und altersmäßig auf das Individuum abgestimmt sind.

11. Verfahren nach einem der vorhergehenden Ansprüche, wobei der Spiegel von mindestens einer Lipidspezies in einer Referenzprobe auf einem computerlesbaren Datenträger gespeichert ist.

12. Verfahren nach einem der vorhergehenden Ansprüche, ferner umfassend Bestimmen des Verhältnisses einfach ungesättigter Fettsäuren (MUFA) gegenüber mehrfach ungesättigter Fettsäuren (PUFA) Lipidspezies, wobei ein höheres Verhältnis von MUFA gegenüber PUFA, verglichen mit dem in der Referenz bestimmten Verhältnis von MUFA gegenüber PUFA, darauf hinweist, dass die Probe von einem Individuum mit einer erhöhten Lebenserwartung stammt.

## Revendications

1. Méthode de typage d'un échantillon provenant d'un individu mammifère, consistant à :

déterminer un taux d'au moins une espèce lipidique dans un échantillon approprié provenant de l'individu, l'au moins une espèce lipidique comprenant des phosphocholines éthérées PC (O-36:2) et/ou PC (O-36:3) ; comparer ledit taux à une référence ; et typer ledit échantillon en tant qu'échantillon d'un individu présentant une espérance de vie accrue si l'échantillon a un taux accru de l'au moins un lipide par comparaison à la référence sur la base de ladite comparaison.

2. Méthode selon la revendication 1, dans laquelle ladite au moins une espèce lipidique comprenant PC (O-36:2) et/ou PC (O-36:3) comprend en outre au moins une des sphingomyélines SM (d18:1/14:0) ; SM (d18:1/15:0) ; SM (d18:1/12:0) ; et/ou SM (d18:1/23:1) et dans laquelle un taux accru de PC (O-36:2), PC (O-36:3), SM (d18:1/14:0) ; SM (d18:1/15:0) ; SM (d18:1/12:0) ; et/ou SM (d18:1/23:1) par comparaison au taux des espèces lipidiques respectives dans la référence, indique que l'échantillon provient d'un individu présentant une espérance de vie accrue.

3. Méthode selon la revendication 2, dans laquelle ladite au moins une espèce lipidique comprend PC (O-36:2), PC (O-36:3), SM (d18:1/14:0) ; SM (d18:1/15:0) ; SM (d18:1/12:0) ; et SM (d18:1/23:1).

4. Méthode selon l'une quelconque des revendications 1 à 3, dans laquelle ladite au moins une espèce lipidique comprend en outre la phosphoéthanolamine (PE) (38:6) et/ou un triglycéride (TG) à chaîne longue et dans laquelle un taux réduit de PE (38:6) et/ou de triglycéride à chaîne longue par comparaison à la référence indique que l'échantillon provient d'un individu présentant une espérance de vie accrue.

5. Méthode selon l'une quelconque des revendications 1 à 4, dans laquelle l'échantillon est un échantillon de sang.

6. Méthode selon l'une quelconque des revendications 1 à 5, dans laquelle l'individu est un être humain.

7. Méthode selon l'une quelconque des revendications 1 à 6, dans laquelle l'individu est une femme.

8. Méthode selon l'une quelconque des revendications 1 à 7, dans laquelle l'individu est d'âge moyen.

9. Méthode selon l'une quelconque des revendications précédentes, dans laquelle la référence est une mesure du taux moyen de ladite au moins une espèce lipidique dans au moins 10 individus indépendants.

10. Méthode selon la revendication 9, dans laquelle lesdits au moins 10 individus indépendants sont du même âge et du même sexe que l'individu.

11. Méthode selon l'une quelconque des revendications précédentes, dans laquelle le taux de ladite au moins une espèce lipidique dans un échantillon de référence est stocké sur un support de données lisible par ordinateur.

12. Méthode selon l'une quelconque des revendications précédentes, consistant en outre à déterminer le rapport des espèces lipidiques acides gras monoinsaturés (MUFA) aux acides gras polyinsaturés (PUFA), un rapport MUFA/PUFA plus élevé, par comparaison au rapport MUFA/PUFA déterminé dans la référence, indiquant que l'échantillon provient d'un individu présentant une espérance de vie accrue.

Figure 1

Figure 2

Figure 3

## REFERENCES CITED IN THE DESCRIPTION

**Patent documents cited in the description**

- WO 2012164525 A

**Non-patent literature cited in the description**

- **QUEHENBERGER et al.** *J Lipid Res,* 2010, vol. 51, 3299-3305
- **WANG et al.** *J Clin Endocrin & Metab,* 2011, vol. 96, 885-893
- **SUGIYAMA ; AGELLON.** *Biochem Cell Biology,* 2012, vol. 90, 124-141
- **NELSON et al.** *American J Epidem,* 2006, vol. 163, 903-912
- **YEBOAH et al.** *Arteriosclerosis, Thrombosis, and Vascular Biology,* 2010, vol. 30, 628-633
- **SUHRE et al.** *PLoS ONE,* 2010, vol. 5, e13953
- **RHEE et al.** *J Clin Invest,* 2011, vol. 121, 1402-1411
- **HAN et al.** *PLoS ONE,* 2011, vol. 6, e21643
- **PIETILAINEN et al.** *PLoS ONE,* 2007, vol. 2, e218
- **GRAESSLER et al.** *PLoS ONE,* 2009, vol. 4, e6261
- **SYSI-AHO et al.** *PLoS Comput Biol.,* 2011, vol. 7, e1002257
- **LAAKSONEN et al.** *PLoS ONE,* 2006, vol. 1, e97
- **BARZILAI et al.** *J Amer Geriat Soc,* 2003, vol. 49, 76-79
- **HEIJMANS et al.** *PLoS Med.,* 2006, vol. 3, e495
- **VAARHORST et al.** *AGE: Journal of the American Aging,* 2011, vol. 33, 219-227
- **CAPRARI et al.** *Experim Geront,* 1999, vol. 34, 47-57
- **PUCA et al.** *Rejuvenation Research,* 2007, vol. 11, 63-72
- **HU et al.** *J Proteome Res,* 2008, vol. 7, 4982-4991
- **BLIGH ; DYER.** *Can J Biochem Physiol.,* 1959, vol. 37, 911-917
- **VAN DER KLOET et al.** *J Proteome Res,* 2009, vol. 8, 5132-5141
- **MOOIJAART et al.** *PLoS Med.,* 2006, vol. 3, e176
- **ROZING et al.** *Aging,* 2009, vol. 1, 714-722
- **WESTENDORP et al.** *J Amer Geriat Soc,* 2009, vol. 57, 1634-1637
- **ROZING et al.** *J Amer Geriat Soc,* 2010, vol. 58, 564-569
- **BLIGH ; DYER.** *Can J Biochem Physiol,* 1959, vol. 37, 911-917
- **VAARHORST et al.** *AGE,* 2011, vol. 33, 219-227
- **PAMPLONA et al.** *Mechanisms Ageing Develop,* 2000, vol. 112, 169-183
- **WESTENDORP.** *J Amer Geriat Soc,* 2009, vol. 57, 1634-1637
- **HEILBRONN et al.** *JAMA,* 2006, vol. 295, 1539-48
- **CURRAN et al.** *Psychological assessment,* 1995, vol. 7, 80-83
- **RADLOFF.** *Applied Psychological Measurement,* 1977, vol. 1, 385-401
- **WARE et al.** *Medical care,* 1996, vol. 34, 220-33
- **FOLSTEIN et al.** *J Psychiatr Res,* 1975, vol. 12, 189-98